# EUROPÄISCHE PATENTANMELDUNG

(11) **EP 4 636 057 A2**
(43) Veröffentlichungstag der Anmeldung: **22.10.2025**
(21) Anmeldenummer: 25200485.8
(22) Anmeldetag: 06.04.2020
(51) Int. Cl.: C10B 53/07

(54) **VERFAHREN ZUM REZYKLIEREN GLASFASERVERSTÄRKTER KUNSTSTOFFE**

(30) Priorität: 15.04.2019 EP 19169215
(62) Teilanmeldung aus: 20715898.1
(71) Anmelder: Envalior Deutschland GmbH, 40474 Düsseldorf (DE)
(72) Erfinder: WITT, Michael, 40474 Düsseldorf (DE); JOACHIMI, Detlev, 40474 Düsseldorf (DE)
(74) Vertreter: Envalior Association

(57) **Zusammenfassung**

Die vorliegende Erfindung betrifft ein Verfahren zum Rezyklieren glasfaserverstärkter Kunststoffe, insbesondere Kunststoffe auf Basis Polybutylenterephthalat oder Polyethylenterephthalat. Das Verfahren zum Rezyklieren von GFK, is gekennzeichnet durch
1. Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
2. Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte
mit der Maßgabe, dass die Polymermatrix eines im erfindungsgemäßen Verfahren einzusetzenden GFK im Wesentlichen wenigstens ein Polymer enthält aus der Gruppe Polyethlenterephthalat (PET), Polybutylenterephthalat (PBT) oder ein Mischpolymer aus PET und PBT.

## Beschreibung

Die vorliegende Erfindung betrifft ein Verfahren zum Rezyklieren glasfaserverstärkter Kunststoffe, insbesondere Kunststoffe auf Basis von Polyamid, Polybutylenterephthalat oder Polyethylenterephthalat, um sowohl Monomere des Polymers, als auch das für die Glasfasern eingesetzte Glas zurück zu gewinnen.

### Stand der Technik

Moderne, hoch belastbare Verbundkunststoffe basieren heute oftmals auf glasfaserverstärkten Thermoplasten, insbesondere Polyamiden oder Polyestern auf Basis der Terephthalsäure. Die LANXESS Deutschland GmbH, Köln, verkauft Kunststoff-Granulate mit Kurzglasfaserverstärkung unter den Handelsnamen Durethan^{®} und Pocan^{®}; endlosfaserverstärkte Halbzeuge bzw. Composites werden unter dem Markennamen TEPEX^{®} angeboten. Der Massenanteil der Glasfaserverstärkung liegt bei den angebotenen Granulaten üblicherweise im Bereich von 5 bis 80 Gewichtsprozent.

Mit Hilfe Glas-basierter Füll- und Verstärkungsstoffe, insbesondere in Form von Fasern, werden erhebliche Verbesserungen bei den Festigkeiten, Zähigkeiten und Steifigkeiten im Vergleich zu einem Kunststoffbauteil ohne Füll- oder Verstärkungsstoff erzielt. Dies ermöglichte in den letzten Jahren die Substitution vieler Metall-basierter Konstruktionen durch Glasfaser-verstärkte Kunststoffe (GFK), insbesondere im Automobilbau.

Zusätzliche thermostabilisierende Additive ermöglichen inzwischen sogar den Einsatz von GFK in thermisch hochbelasteten Bereichen, die für das reine, nicht mit diesen Additiven stabilisierte Polymer, unmöglich wären, insbesondere im Bereich von Kraftfahrzeug-Motorräumen.

GFK basierte Bauteile ermöglichen heute einen kostengünstigen Leichtbau im gesamten Transportsektor. Durch die erzielte Gewichtsreduktion bei Kraftfahrzeugen können die Energieverbräuche (Kraftstoff oder elektrische Energie) signifikant reduziert werden.

Am Ende einer Nutzungsperiode, die im Falle eines Kraftfahrzeugs mit dessen Abgabe bei einem Autoverwerter endet, stellen GFK basierte Bauteile jedoch sehr hohe Anforderungen an ein Recycling, insbesondere wenn beabsichtigt wird, stoffgleiches Neumaterial zu ersetzen. Dies trifft umso mehr für GFK-Bauteile zu, die für die Zielanwendung hoch additiviert werden müssen, um während des Gebrauchs ein Downgrading zu vermeiden bzw. zu reduzieren. Unter Downgrading ist im Sinne der vorliegenden Erfindung eine Verschlechterung des mechanischen Eigenschaftsniveaus zu verstehen, insbesondere durch Spaltung der Molekülketten des (Matrix)Polymers.

Für das Qualitätsniveau von GFK basierten Kunststoff-Rezyklaten spielen auch Verunreinigungen eine wesentliche Rolle. Ein werkstoffliches Recycling erfordert dann besonders hohe Anforderungen an den zu rezyklierenden Kunststoffabfall, wenn das Rezyklat zur Herstellung derselben oder für andere anspruchsvolle Anwendungen verwendet werden soll. Bei dem werkstofflichen Recycling werden nur physikalische Prozesse verwendet. Physikalische Prozesse sind beispielsweise Waschen, Trocknen, Zerkleinern, Aufschmelzen, Compoundieren, Schmelzefiltration und erneutes Granulieren. Aber natürlich gibt es Beispiele, wo Kunststoffabfall über physikalische Prozesse zu Rezyklat mit guter Qualität rezykliert werden kann. Meist sind folgende Voraussetzungen notwendig:
Die Bauteile aus GFK müssen sortenrein gesammelt werden, der Verschmutzungsgrad sollte gering sein, während der Nutzungsperiode sollte kein deutlicher Polymerabbau stattgefunden haben und zudem sollten über den Verlauf der gesamten Nutzungsperiode nur wenig Fremdstoffe von der Polymermatrix absorbiert worden sein, beispielsweise Spezialöle nebst deren Additivierungen, beispielsweise bei Motor- und Getriebeölwannen, beispielsweise für PKW oder LKW, oder Kühlmittel im Falle von Kühlkreislaufbauteilen wie Kühlerwasserkästen. Mit Hilfe üblicher Spritzgussmaschinen können solche Kunststoffbasierten Rezyklat-Granulate wieder zur Herstellung neuer Bauteile genutzt werden.

Es hat sich jedoch gezeigt, dass ein werkstoffliches Recycling allein über physikalische Verfahren in den seltensten Fällen zu einem Qualitätsniveau führt, das dem der ursprünglichen Neuware entspricht.

Folgende Aspekte kommen bei GFK erschwerend hinzu, die ein Downgrading begünstigen und damit ein werkstoffliches Recycling, insbesondere allein über physikalische Verfahren, erschweren:
- Ein hoher Gehalt von Kurzglasfasern verhindert beim Compoundieren den Einsatz von Schmelzefiltern, die, insbesondere bei kontinuierlicher Abreinigung und Ausschleusung abgeschiedener Verunreinigungen, beim werkstofflichen Recycling ungefüllter Thermoplaste einen entscheidenden Beitrag zur Qualitätsverbesserung der gewonnenen Rezyklate beitragen.
- Bei der Compoundierung, vorzugsweise mit den üblicherweise dabei verwendeten gleichdrehenden Doppelschneckenextrudern, werden Glasfasern mechanisch verkürzt, ein Effekt, der direkt die Festigkeit und Zähigkeit des GFK bzw. Compounds bzw. Composites negativ beeinflusst.
- Additive, insbesondere Flammschutzmittel oder Thermostabilisatoren, unterliegen während der Nutzungsperiode eines GFK, insbesondere bei hohen Dauergebrauchstemperaturen, einer Veränderung. Die Abbauprodukte solcher Additive verbleiben aber im Kreislauf des werkstofflichen Recyclings und damit im GFK-Rezyklat.

Natürlich gibt es beim werkstofflichen Recycling von GFK mit Hilfe physikalischer Methoden eine Vielzahl von Vorschlägen, dem Downgrading entgegenzuwirken. Beispielsweise kann der Effekt einer im Rezyklat reduzierten Faserlänge durch eine Nachdosierung längerer Fasern ausgeglichen werden. Dieses Verfahren ist aber naturgemäß für endlosfaserverstärkte Composite Materialien nicht geeignet und daher nur auf kurzglasfaserverstärkte GFK beschränkt.

Auch dem Polymerabbau kann auf vielfältige Weise über gezielte Additivierung, insbesondere über chemisch aktivierte Kettenverlängerung, entgegengewirkt werden.

Es bleibt aber festzuhalten, dass das Downgrading ein prinzipielles Problem beim werkstofflichen Recycling von GFK darstellt und ein mehrmaliges Durchlaufen der Verwendungs-Recycling-Kreisläufe bei GFK nicht ohne deutliche Beeinflussung der Qualität und der Produkteigenschaften, insbesondere bezüglich Zähigkeit, Festigkeit, Steifigkeit, Kriechen, Wärmeformbeständigkeit usw., möglich ist.

Wie erwähnt, nimmt die Faserlänge beim erneuten Compoundieren ab. Zudem ist es im Allgemeinen nicht möglich, die Kurzglasfasern vom im geschmolzenem Zustand hochviskosen (Matrix)Polymer direkt abzutrennen und somit die Polymermatrix von den Fasern zu separieren.

Dem hohen Aufwand, die Fasern von der Matrix zu separieren, ggf. weiter zu reinigen und für eine Verwendung als Rezyklatfasern vorzubereiten, steht letztlich auch der günstige Preis für Glasfaser-Neuware gegenüber.

Alle drei Gründe erklären, warum die Glasfasern aus GFK-Altmaterialien bisher nur in seltenen Fällen rezykliert und einer erneuten Nutzung als Füll- oder Verstärkungsstoff zugeführt werden.

WO 2017 007965 A1 beschreibt ein Verfahren zur Depolymerisation von unverstärktem Polyethylenterephthalat um daraus Terephthalsäure und Ethylenglykol zu gewinnen. Hierzu wird das Polymer in eine Mischung aus einem unpolaren, das Polymer quellenden Lösungsmittel und einem die Esterfunktionalität aufbrechenden Reagens gegeben und depolymerisiert. Auf das Recycling von Füll- oder Verstärkungsstoffen, insbesondere von Glasfasern geht WO 2017 007965 A1 nicht ein.

Die EP 3 023 478 A2 offenbart ein Verfahren, das es erlaubt, speziell bei Kohlefaser-Verbundkunststoffen die Fasern zurückzugewinnen. Dabei wird die Polymermatrix des Verbundkunststoffs in einem Hauptreaktor zunächst bei 400-600°C pyrolysiert. Der verbleibende Faser-Rückstand mit Rußrückständen wird gewaschen, wobei die Fasern Wasser adsorbieren. Anschließend wird der feuchte Rückstand erneut in den Hauptreaktor überführt, wobei bei 350-400°C durch oxidierende Bedingungen die Fasern gereinigt werden. Die im ersten Schritt bei der Pyrolyse entstehenden Spaltprodukte werden stofflich nicht verwertet, sondern in einen zweiten Reaktor überführt, wo mit Hilfe eines thermischen Plasmas bei bis zu 15.000°C die toxikologischen Spaltprodukte neutralisiert werden.

**Die** JP 2000034363A beschreibt ein Verfahren zur Depolymerisation von Kurzglasfaserverstärkten Polyamid 6 Verbundkunststoffen, bei dem zunächst die Polymermatrix bei Temperaturen um 280°C depolymerisiert wird, dann die gesamte Rektionsmischung in Wasser gegeben wird und das durch Depolymerisation erhaltene Caprolactam - also der Monomerbaustein des Polyamid 6 - in Wasser gelöst wird. Aufgrund der deutlich niedrigeren Viskosität der wässrigen Caprolactam-Lösung, die oft nur 1 - 100 mPa•s beträgt, können hier die Glasfasern von der kontinuierlichen Wasserphase abgetrennt und gewaschen werden.

Nachteilig am Verfahren der JP 2000034363A ist die energieaufwändige Destillation zur Gewinnung hochreinen Caprolactams aus den wässrigen, verdünnten CaprolactamLösungen. Auch die Wiederverwendung der in JP 2000034363A abgetrennten und gewaschenen Glasfasern ist nicht unproblematisch. Durch die im Verarbeitungsprozess erfolgte Verkürzung der Faserlängen lässt sich mit den abgetrennten und gewaschenen Glasfasern aus JP 2000034363A nicht mehr der gleiche verstärkende Effekt wie bei Verwendung von Glasfaser-Neuware erzielen. Zudem enthalten Verbundkunststoffe heute eine Vielzahl an Additiven, die auf den Fasern zurückbleiben und auch nicht vollständig mit Wasser abgewaschen werden können. Das Verfahren nach JP 2000034363A liefert in diesen Fällen dann auch keine hochwertigen Rezyklatfasern.

Schließlich benötigen glasbasierte Verstärkungsfasern für eine optimale Funktion eine gute Verträglichkeit der Glasfaser-Oberfläche mit der Polymermatrix, die üblicherweise über maßgeschneiderte Oberflächenbeschichtungen, auch als Schlichten bezeichnet, erzielt wird. Das Aufbringen einer geeigneten Schlichte auf die getrockneten, rezyklierten Glasfaseragglomerate, wie sie nach JP 2000034363A erhalten werden, erwies sich als nicht durchführbar: Die mit wässriger Schlichte behandelten Faseragglomerate konnten nach dem Trocknen nicht erneut separiert und in eine dosierfähige Form gebracht werden. In keinem Fall konnte mit Hilfe des in JP 2000034363A beschriebenen Verfahrens die Qualität von Glasfaser-Neuware erzielt werden.

Auch JP2000037726A beschreibt ein Verfahren zur Abtrennung von Glasfasern, hier Kurzglasfasern, bei der Rezyklierung von Polyamid 6 (PA6) basierten Verbundkunststoffen. Gemäß JP2000037726A wird zunächst die PA6-Polymermatrix depolymerisiert und somit von den Glasfasern abgetrennt.JP2000037726A beschreibt zusätzlich die prinzipielle Möglichkeit, auch die Glasfasern zurück zu gewinnen, indem am Ende der Depolymerisation durch Erhitzen auf 400° - 700°C auf den Fasern verbliebene Restbestandteile des als Matrixpolymer eingesetzten PA6 mittels pyrolytischer Zersetzung in gasförmige Bestandteileüberführt werden. Die zur Pyrolyse notwendige Temperatur muss dem Prozess von außen zugeführt werden. JP 2000037726A beschreibt schließlich die Option, die auf diese Weise "aufgereinigten Fasern" anschließend auf Temperaturen über deren Schmelztemperatur zu erwärmen. Auch diese zum Schmelzen der Glasfasern erforderliche Energie muss extern zugeführt werden.

Weder JP 2000034363A noch JP2000037726A befassen sich mit der grundsätzlichen Problematik der zusätzlich im Kunststoff eingesetzten Additive, mit deren Abbauprodukten sowie mit deren Entfernung aus dem Waschwasser oder den Pyrolyse-Produkten. Letzteres ist aber erforderlich, um diese oft für die Umwelt nicht unbedenklichen Stoffe nicht in die Umwelt gelangen zu lassen.

### Aufgabe der vorliegenden Erfindung

Ausgehend vom oben beschriebenen Stand der Technik bestand die Aufgabe der vorliegenden Erfindung darin, ein Verfahren zum Rezyklieren von GFK, vorzugsweise von Polyamid 6, Polybutylenterephthalat (PBT) und Polyethylenterephthalat (PET) basierten GFK, ohne den Einsatz von (Wasch)Wasser zur Reinigung der Glasfasern bereit zu stellen, womit sowohl das (Matrix)Polymer in Form seiner Monomere, als auch die Glasfasern in Form von für die Glasfaserherstellung geeignetem Glas zurückgewonnen und im Polymerisationsprozess bzw. dem Glasfaserherstellungsprozess zu Polymer bzw. Glasfaser mit Neuwarequalität verarbeitet werden können. Dabei sollen gleichzeitig Additive effektiv und ohne Beeinträchtigung der Umwelt aus dem Rezyklierungs-Kreislauf ausgeschleust werden können und das Verfahren so wirtschaftlich sein, dass eine großtechnische Umsetzung auch ökonomisch sinnvoll ist.

Überraschenderweise wurde gefunden, dass es im Gegensatz zur Lehre des oben zitierten Stands der Technik möglich ist, aus GFK-Altmaterialien, insbesondere aus Glasfaserverstärkten Thermoplasten basierend auf PA6, PET oder PBT, Glasfasern in Neuwarenqualität herzustellen und zudem einen Großteil der Polymermatrix über Depolymerisation abzutrennen, welches wieder zu dem gleichen, ursprünglichen Polymer aufgebaut werden kann.

### Gegenstand der Erfindung

Die Lösung der Aufgabe und damit Gegenstand der vorliegenden Erfindung ist ein Verfahren zum Rezyklieren von GFK durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile anreichert und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte.

Die Polymermatrix bzw. deren Spaltprodukte werden folglich im Verfahrensschritt a) nicht quantitativ von den Glasfasern abgetrennt.

Erfindungsgemäß "problematische Bestandteile" sind insbesondere Verunreinigungen sowie für den ursprünglichen Einsatzzweck des GFK eingesetzte Additive.

Für das erfindungsgemäße Recycling von GFKs problematische Bestandteile sind vorzugsweise Funktionsadditive, insbesondere UV-Stabilisatoren, Thermostabilisatoren, Gammastrahlenstabilisatoren, Antistatika, Elastomermodifikatoren, Fließhilfsmittel, Entformungsmittel, Flammschutzmittel, Emulgatoren, Nukleierungsmittel, Weichmacher, Gleitmittel, Farbstoffe, Pigmente oder Additive zur Erhöhung der elektrischen Leitfähigkeit. Die genannten und weitere Additive sind zum Beispiel beschrieben in Gächter, Müller, Kunststoff-Additive, 3. Ausgabe, Hanser-Verlag, München, Wien, 1989 **und im** Plastics Additives Handbook, 5th Edition, Hanser-Verlag, München, 2001**.** Verunreinigungen im Sinne der vorliegenden Erfindung sind vorzugsweise Abbauprodukte der Additive sowie Verschmutzungen, insbesondere Staub, Erden, Eisenoxide in Form von Rost oder in die Polymermatrix eingedrungene oder an dieser anhaftende Fremdstoffe.

Das erfindungsgemäße Verfahren besteht damit aus zwei separaten Prozessen, bei denen die ursprünglich zur Herstellung von GFK eingesetzten Komponenten einerseits in Monomere und Spaltprodukte und andererseits in eine Glasschmelze getrennt werden.

Das erfindungsgemäße Verfahren gestattet es zudem, die für ein Recycling problematischen Bestandteile, insbesondere Verunreinigungen, zu entfernen und damit Rezyklate mit Neuwarencharakter zu erzeugen.

Beim erfindungsgemäßen Verfahren findet selbst nach dem Durchlaufen mehrerer Nutzungs-Rezyklierungszyklen keine wesentliche Beeinträchtigung der Produktqualität des dem GFK zugrunde liegenden (Matrix)Polymer statt, wenn dieses aus den als Spaltprodukten anfallenden Monomeren wieder synthetisiert wird. Nach dem erfindungsgemäßen Verfahren wird die Polymermatrix bzw. das zu rezyklierende (Matrix)Polymer zu über 50 Gew.-% bis zu etwa 80 Gew.-% rezykliert.

Zur Klarstellung sei angemerkt, dass vom Rahmen dieser Erfindung alle aufgeführten, allgemeinen oder in Vorzugsbereichen genannten Definitionen und Parameter in beliebigen Kombinationen umfasst sind. Dies betrifft sowohl stoffliche Parameter, als auch jegliche Formen der Verwendung und Verfahren, wie sie im Rahmen der vorliegenden Erfindung beschrieben werden. Sofern nicht anders angegeben gelten zitierte Normen in der zum Anmeldetag gültigen Fassung. Sofern nicht anders angegeben handelt es sich bei Prozentangaben um Gewichtsprozente. Im Rahmen der vorliegenden Erfindung haben die Begriffe (Matrix)Polymer und Polymermatrix dieselbe Bedeutung, wobei der Fokus/die Betonung beim Begriff Polymermatrix auf der Matrix liegt, während beim Begriff (Matrix)Polymer die Betonung auf dem Polymer liegt.

Gemäß **Kunststoffe.de, "Begriffsdefinitionen für das werkstoffliche Recycling", Auszug aus W. Hellerich, G.Harsch, E.Baur, Werkstoff-Führer Kunststoffe** 10/2010, S. 55 unter:
**https://www.kunststoffe.de/themen/basics/recycling/werkstofflichesrecycling/artikel/begriffsdefinitionen-fuer-das-werkstoffliche-recycling-1001597.html** ist **Rezyklat** ein Überbegriff, wobei es sich um eine Formmasse bzw. einen aufbereiteten Kunststoff mit definierten Eigenschaften handelt. In vielen Fällen wird das Rezyklat in Neuware eingemischt. Ein Rezyklat hat in seinem Werdegang i.a. bereits einen Verarbeitungsprozess hinter sich. Ein Masterbatch oder ein Blend, die aus mehreren Kunststoffen durch Aufbereiten, also durch einen Verarbeitungsprozess hergestellt wurden, gelten nicht als Rezyklate.

### Bevorzugte Ausführungsformen der Erfindung

Vorzugsweise betrifft die vorliegende Erfindung ein Verfahren, bei dem der in Verfahrensschritt a) einzusetzende GFK erhitzt und gegebenenfalls nach Zugabe von Katalysatoren oder die Depolymerisation fördernden Hilfsstoffe unter Luftausschluss die Polymermatrix des GFK gespalten wird.

Vorzugsweise betrifft die vorliegende Erfindung deshalb ein Verfahren, bei dem während oder im Anschluss an Verfahrensschritt a) durch Reduzierung des Druckes die Spaltprodukte und/oder die zugesetzten Hilfsstoffe, insbesondere Hydrolyse- oder Solvolyse-Flüssigkeiten, abdestilliert werden.

Vorzugsweise betrifft die vorliegende Erfindung ein Verfahren zum Rezyklieren von GFK, bei dem im Verfahrensschritt a) mindestens 50 Gew.% der Polymermatrix depolymerisiert werden.

Besonders bevorzugt betrifft die vorliegende Erfindung ein Verfahren zum Rezyklieren von GFK, bei dem im Verfahrensschritt a) mindestens 50 Gew.-% und höchstens 80 Gew.-% der Polymermatrix depolymerisiert werden.

Im Falle, dass Verfahrensschritt b) nicht in einem Ofen zur Glasproduktion durchgeführt wird, erfolgt nach Verfahrensschritt b) in einem weiteren Verfahrensschritt c) die Abtrennung der Glasschmelze für eine Weiterverarbeitung.

### Verfahrensschritt a)

Vorzugsweise wird die Depolymerisation im Verfahrensschritt a) unter Zusatz von Hilfsstoffen oder Katalysatoren durchgeführt, um die Depolymerisation zu fördern. Bevorzugte, die Depolymerisation von (Matrix)Polymeren fördernde Katalysatoren sind Basen oder Säuren oder deren Salze. Besonders bevorzugt sind anorganische Basen oder anorganische Säuren oder deren Salze. Ganz besonders bevorzugt wird Calciumhydroxid, Calciumcarbonat, Natriumcarbonat, Kaliumcarbonat oder Phosphorsäure eingesetzt. Diese, die Depolymerisation von (Matrix)Polymeren fördernden Katalysatoren, werden in Konzentrationen im Bereich von 0,1 bis 20 Gew.-%, bevorzugt in Konzentrationen im Bereich von 0,5 bis 10 Gew.-%, besonders bevorzugt im Bereich von 1 bis 7 Gew.-%, eingesetzt, jeweils bezogen auf die insgesamt in Verfahrensschritt a) eingetragene Polymermatrix.

Vorzugsweise enthält die Polymermatrix eines im erfindungsgemäßen Verfahren einzusetzenden GFK im Wesentlichen wenigstens ein Polymer aus der Gruppe Polyamid 6 (PA6), Polyamid 66 (PA66), Polyethlenterephthalat (PET), Polybutylenterephthalat (PBT) oder ein Mischpolymer aus PET und PBT. Im Wesentlichen bedeutet vorzugsweise zu wenigstens 70 Gew.-%, bezogen auf die in Verfahrensschritt a) eingetragene Polymermatrix.

Vorzugsweise enthält die Polymermatrix eines im erfindungsgemäßen Verfahren einzusetzenden GFK im Wesentlichen Polyamid 6 (PA6), Polyamid 66 (PA66), Polybutylenterephthalat (PBT) oder ein Mischpolymer aus PBT und PET.

Vorzugsweise enthält die Polymermatrix eines im erfindungsgemäßen Verfahren einzusetzenden GFK im Wesentlichen Polyamid 6 (PA6) oder Polyamid 66 (PA66).

Vorzugsweise enthält die Polymermatrix eines im erfindungsgemäßen Verfahren einzusetzenden GFK im Wesentlichen Polybutylenterephthalat (PBT) oder ein Mischpolymer aus PBT und PET.

Im Falle des PA6 wird als Depolymerisationsprodukt im Verfahrensschritt a) ε-Caprolactam gewonnen. Vorzugsweise lassen sich durch Depolymerisation in Verfahrensschritt a) aus PA6 50 bis 80 Gew.-% des zu dessen Herstellung ursprünglich eingesetzten ε-Caprolactams wiedergewinnen.

Im Rahmen der Arbeiten zur vorliegenden Erfindung wurde gefunden, dass zu Beginn einer in Verfahrensschritt a) stattfindenden Depolymerisation von GFK auf Basis von Glasfaserverstärktem PA6 hohe Zersetzungsraten auftreten und sich wenige Fremdstoffe in den Spaltprodukten befinden. Bevorzugtes Spaltprodukt bei der in Verfahrensschritt a) erfolgenden Depolymerisation von PA6 ist erfindungsgemäß ε-Caprolactam. Insbesondere führen Kaliumcarbonat oder Natriumcarbonat zu hohen Ausbeuten an ε-Caprolactam.

Vorzugsweise werden die in Verfahrensschritt a) einzusetzenden GFK Komponenten vor dem Einsatz in der Depolymerisation **sortenähnlich** gesammelt und auch sortenähnlich im Verfahrensschritt a) eingesetzt. Sortenähnlich bedeutet, dass die aufzubereitenden Kunststoffe zwar in ihren Grundpolymeren übereinstimmen, aber in besonderen Eigenschaften, z.B. flammhemmende Zusätze, voneinander abweichen. Siehe: **Kunststoffe.de, "Begriffsdefinitionen für das werkstoffliche Recycling", Auszug aus W. Hellerich, G.Harsch, E.Baur, Werkstoff-Führer Kunststoffe 10/2010, S. 55** unter:
**https://www.kunststoffe.de/themen/basics/recycling/werkstofflichesrecycling/artikel/begriffsdefinitionen-fuer-das-werkstoffliche-recycling-1001597.html**

Besonders bevorzugt werden die in Verfahrensschritt a) einzusetzenden GFK Komponenten vor dem Einsatz in der Depolymerisation **sortenrein** gesammelt und auch sortenrein im Verfahrensschritt a) eingesetzt, damit die in Verfahrensschritt a) abgetrennten Spaltprodukte keiner aufwändigen Aufarbeitung unterzogen werden müssen.

Sortenrein im Sinne der vorliegenden Erfindung bedeutet, dass Kunststoffe mit gleicher Kennzeichnung nach **DIN EN ISO 11469** bzw. **VDA 260,** ggf. verschiedener Rohstoffhersteller aufbereitet werden. Siehe: **Kunststoffe.de, "Begriffsdefinitionen für das werkstoffliche Recycling", Auszug aus W. Hellerich, G.Harsch, E.Baur, Werkstoff-Führer Kunststoffe 10/2010, S. 55** unter:
**https://www.kunststoffe.de/themen/basics/recycling/werkstofflichesrecycling/artikel/begriffsdefinitionen-fuer-das-werkstoffliche-recycling-1001597.html**

Vorzugsweise werden die in Verfahrensschritt a) einzusetzenden GFK Komponenten vor Einsatz in der Depolymerisation einer Reinigung unterzogen, um anhaftende Verunreinigungen erst gar nicht in die Pyrolyse des Verfahrensschrittes a) einzuführen.

Vorzugsweise werden die in Verfahrensschritt a) einzusetzenden GFK Komponenten vor Einsatz in der Depolymerisation zu Kleinteilen geschreddert, um die Handhabung, Förderbarkeit und Depolymerisation des (Matrix)Polymers zu vereinfachen bzw. zu beschleunigen. Schreddern steht im Rahmen der vorliegenden Erfindung repräsentativ für jegliche Zerkleinerungsverfahren, insbesondere mechanische Zerkleinerungsverfahren. Erfindungsgemäß dem Verfahrensschritt a) vorgeschaltete Zerkleinerungsverfahren wurden beispielsweise im Projekt **Recycling von Polymeren aus Schredderfraktionen,** Projektpartner UNISENSOR Sensorsysteme GmbH, Karlsruhe wissenschaftlich untersucht. Die aus dem Projekt hervorgegangene DE 10 2014 111871 B1 betrifft eine Vorrichtung und ein entsprechendes Verfahren zur Trennung einer oder mehrerer Materialfraktionen aus mindestens einem Materialstrom rieselfahigen Schüttgutes, vorzugsweise aus Bruchstücken recyclingfähiger Kunststoffe. Der Inhalt dieser DE 10 2014 111871 B1 wird von der vorliegenden Anmeldung vollumfänglich umfasst.

In einer weiteren bevorzugten Variante wird die Schredderfraktion vor der Depolymerisation in Verfahrensschritt a) zu Partikeln mit Partikelgrößen < 10 mm, besonders bevorzugt < 5 mm, vermahlen. Der in diesem Zusammenhang verwendete Begriff Mahlgut, das durch Mahlen von Kunststoff gewonnen wird, hat insbesondere bevorzugt unterschiedliche und unregelmäßige Teilchengrößen im Bereich von 2 bis 5 mm und kann Staubanteile enthalten. Siehe hierzu: **Kunststoffe.de, "Begriffsdefinitionen für das werkstoffliche Recycling", Auszug aus W. Hellerich, G.Harsch, E.Baur, Werkstoff-Führer Kunststoffe 10/2010, S. 55** unter:
**https://www.kunststoffe.de/themen/basics/recycling/werkstofflichesrecycling/artikel/begriffsdefinitionen-fuer-das-werkstoffliche-recycling-1001597.html**

In einer weiteren bevorzugten Variante wird die Schredderfraktion vor der Depolymerisation in Verfahrensschritt a) zu Pulver mit Partikelgrößen < 1 mm vermahlen und dabei das Pulver mit wenigstens einem, die Depolymerisation fördernden Hilfsstoff und/oder Katalysator gemischt.

In einer Ausführungsform kann bei Bedarf die beim Schreddern anfallende Fraktion (Schredderfraktion) vor dem Einsatz in der Depolymerisation in Verfahrensschritt a) weiter aufgearbeitet werden. Vorzugsweise können dabei mit Hilfe zusätzlicher Verfahrensschritte am (Matrix)Polymer anhaftende Metallkomponenten entfernt werden und separat rezykliert werden. Bevorzugt werden in diesem Fall Magnetabscheider oder Induktionsabscheider eingesetzt.

Die Wahl des wenigstens einen, die Depolymerisation fördernden Katalysators und/oder Hilfsstoffes erfolgt dabei bevorzugt in der Weise, dass diese im Verfahrensschritt b) unter Energiegewinnung rückstandslos verbrennen, oder als anorganische Bestandteile in der Glaskomponente und schließlich im zurückgewonnenen Glas verbleiben können, ohne die Glasqualität merklich zu beeinflussen.

Vorzugsweise verbleiben in Verfahrensschritt a) wenigstens 20 Gew.-% der ursprünglichen Polymermatrix als organischer Rückstand. Diese verbleibenden wenigstens 20 Gew.-% Polymermatrix werden in Verfahrensschritt b) genutzt, um über den Verbrennungsprozess und der dabei entstehenden Verbrennungswärme die Glas basierte Komponente, vorzugsweise Glasfasern, aufzuschmelzen.

Vorzugsweise wird dabei die Glas basierte Komponente gleichzeitig von organischen Verunreinigungen befreit. Vorzugsweise werden in dem Verfahrensschritt a) GFK Komponenten auf Basis einfach und schnell depolymerisierbarer Polymere eingesetzt.

Bevorzugte einfach und schnell depolymerisierbare Polymere im Sinne der vorliegenden Erfindung sind Polybutylenterephthalat (PBT), Polyethylenterephthalat (PET) oder Polyamid 6 (PA6).

Im Falle PA6 basierter GFK-Anteile wird vorzugsweise die Depolymerisation durch Erwärmen unter Sauerstoffausschluss, besonders bevorzugt in Gegenwart wenigstens eines basischen Katalysators bei Temperaturen <350°C durchgeführt; in diesem Fall kann die Depolymerisation als Pyrolyse bezeichnet werden.

Im Falle PBT basierter zu recyclierender GFKs wird vorzugsweise die Depolymerisation im Verfahrensschritt a) in Gegenwart von Wasser als die Depolymerisation fördernder Hilfsstoff durchgeführt. Vorzugsweise wird die Depolymerisation PBT basierter GFK bei Temperaturen im Bereich von 240 bis 350°C durchgeführt. Dabei wird Terephthalsäure und 1,4 Butandiol bzw. dessen Dehydrationsprodukt Tetrahydrofuran gebildet. Es ist ebenfalls möglich, die Depolymerisation in Gegenwart von Alkoholen als Hilfsstoffe in Form einer Solvolyse durchzuführen, was zur Bildung der entsprechenden Ester führt.

Im Falle PET basierter zu recyclierender GFKs wird vorzugsweise die Depolymerisation in Gegenwart von Wasser und/oder Alkoholen bei Temperaturen oberhalb von 280°C durchgeführt.

Vorzugsweise und in einer Ausführungsform werden die in dem Verfahrensschritt a) unter Spaltung der Polymerketten anfallenden Spaltprodukte des GFK (Matrix)Polymers, vorzugsweise nach erforderlicher Aufreinigung, insbesondere durch Destillation, einer sich anschließenden erneuten Polymerisation zur Herstellung von Rezyklat-Kunststoff mit Neuwarencharakter zugeführt. Durch eine erneute Polymerisation der Spaltprodukte ist es möglich, insbesondere nach zusätzlicher Aufreinigung dieser als Monomere bezeichneten Spaltprodukte, wieder das gleiche Polymer bzw. den gleichen Kunststoff, insbesondere zur Herstellung neuer GFK auf Basis von Rezyklat, herzustellen. Diese Verfahrensvariante wird vorzugsweise in den Fällen angewandt, in denen das GFK (Matrix)Polymer nur wenige, im Idealfall nur ein Monomer enthält und sich das oder die gewonnenen Monomere durch im industriellen Großmaßstab etablierte Verfahren, vorzugsweise durch Destillationen oder Rektifikationen, auftrennen und aufreinigen lassen.

Insbesondere bevorzugt nach dem erfindungsgemäßen Verfahren aufzuarbeitende GFKs sind solche auf Basis von PA6 als (Matrix)Polymer, das wiederum auf Basis von ε-Caprolactam als Monomer basiert.

Gegebenenfalls oder in einer bevorzugten Ausführungsform werden die in Verfahrensschritt a) anfallenden Monomere bzw. Spaltprodukte vor einer erneuten Polymerisation zur Herstellung von Rezyklat-Kunststoff großtechnischen Aufbereitungsanlagen zugeführt und zusammen mit klassisch petrochemisch hergestellten Monomeren aufgereinigt. Bevorzugte großtechnische Aufbereitungsanlagen sind auch in diesem Fall Destillationsanlagen oder Rektifizieranlagen.

Vorzugsweise und in einer weiteren Ausführungsform werden Anteile der in Verfahrensschritt a) unter Spaltung der Polymerketten anfallenden Monomere bzw. Spaltprodukte des GFK (Matrix)Polymers auch als zusätzlicher Brennstoff für den Verbrennungsvorgang im Verfahrensschritt b) eingesetzt. Diese Verfahrensvariante wird vorzugsweise in Fällen angewandt, in denen das GFK (Matrix)Polymer aus wenigstens zwei verschiedenen Monomeren aufgebaut ist, oder die Polymermatrix aus einem Blend von mindestens zwei verschiedenen Kunststoffen besteht, oder aber ein sortenreiner Altkunststoff als Feed-Strom nicht vorliegt. Feed-Strom ist ein in der Verfahrenstechnik etablierter Begriff. Man bezeichnet damit einen Zustrom (Zufütterung) an Edukten in ein Verfahren, im vorliegenden Fall das erfindungsgemäße Verfahren zum Rezyklieren von GFK.

Die Depolymerisation, worunter je nach Art des zugrundeliegenden (Matrix)Polymers vorzugsweise eine Hydrolyse, Solvolyse oder Pyrolyse bzw. Thermolyse zu verstehen ist, kann in verschiedenen verfahrenstechnischen Apparaten durchgeführt werden.

Erfindungsgemäß einzusetzende GFKs, insbesondere Polyamid basierte GFKs, werden vorzugsweise nach Zugabe geeigneter Hilfsstoffe bzw. Katalysatoren, insbesondere basischer Katalysatoren, direkt unter Sauerstoffausschluss, vorzugsweise unter Stickstoffatmosphäre, erhitzt (Pyrolyse/Thermolyse). Hierzu werden vorzugsweise Batchreaktoren verwendet, die durch zeitlich versetztes Anfahren quasi kontinuierlich Material für den zweiten Verfahrensschritt b) zur Verfügung stellen.

In den Fällen, in denen über Heißdampf oder mit Hilfe von Alkoholen das (Matrix)Polymer in Verfahrensschritt a) depolymerisiert wird, insbesondere PBT oder PET, werden vorzugsweise Druckautoklaven eingesetzt. Nach einer Einwirkungszeit werden die flüchtigen Komponenten, vorzugsweise unter Druckverminderung, abdestilliert und der verbleibende Rückstand in den Verfahrensschritt b) überführt.

### Verfahrensschritt b)

Vorzugsweise wird Verfahrensschritt b) in einem Drehrohrofen durchgeführt- siehe U. Richers, Thermische Behandlung von Abfällen in Drehrohröfen, Forschungszentrum Karlsruhe GmbH, Karlsruhe, 1995**.**

Vorzugsweise verbleiben in Verfahrensschritt a) maximal 20 Gew.-% des ursprünglichen (Matrix)Polymers als organischer Rückstand, der zusammen mit der Glas basierten Komponente dem Verfahrensschritt b) zugeführt wird. Dieser organische Rückstand wird im Verfahrensschritt b) verbrannt, wobei durch die Verbrennungswärme die Glas basierte Komponente zunächst erhitzt und schließlich aufgeschmolzen wird. Vorzugsweise wird Verfahrensschritt b) bei Temperaturen im Bereich von 1300°C +/- 300°C durchgeführt.

Durch Verbrennung des verbleibenden organischen Materials werden gleichzeitig die auf der Glas basierten Komponente verbliebenen Verunreinigungen, Additive und Zersetzungsprodukte entfernt. Vorzugsweise erfolgt dabei eine Oxidation organischer Verunreinigungen, Additive und Zersetzungsprodukte zu CO₂ und Wasser.

Vorzugsweise wird die gesamte Energie für den Verfahrensschritt b) aus der Verbrennung des mit der Glas basierten Komponente in Verfahrensschritt b) eingetragenen organischen Materials/Rückstands erzeugt.

In einer Ausführungsform wird allerdings in Verfahrensschritt b) zusätzliche Energie zugeführt, bevorzugt mittels konventioneller Gasbrenner. Diesen werden in bevorzugter Ausführungsform als Brennmaterial C₁-C₄-Kohlenwasserstoff basierte gasförmige Brennstoffe zugeführt. Vorzugsweise werden dazu Erdgas oder Biogas eingesetzt.

Die Zuführung zusätzlicher Energie in Verfahrensschritt b) ist vorzugsweise anzuwenden, wenn die Verbrennungswärme des organischen Materials/Rückstands oder des im organischen Rückstand noch enthaltenden Polymers nicht ausreicht, die Schmelztemperatur der Glas basierten Komponente zu erzielen und/oder um die übliche Verweilzeit der Glas basierten Komponente im geschmolzenen Zustand bis zur Weiterverarbeitung zu überbrücken.

Vorzugsweise erfolgt die Verbrennung des organischen Materials/Rückstands in Verfahrensschritt b) unter Zuführung von Luft, Luft-Sauerstoff Gemischen oder reinem Sauerstoff.

Bevorzugt wird der Verfahrensschritt b) direkt im Aufschmelzbereich einer Glasfaserproduktionsanlage durchgeführt, wobei das organische Material bzw. der organische Rückstand über zusätzlich eingespeiste Luft bzw. Sauerstoff verbrannt werden.

Hierbei wird der in Verfahrensschritt a) erhaltene Rückstand als Seitenstrom zum Hauptdosierstrom der anorganischen Glas-Rohstoffmischung in den Aufschmelzbereich des Ofens der Glasfaserproduktionsanlage dosiert, oder gegebenenfalls als Feststoff nach Zerkleinerung, insbesondere Pulverisierung, dem Ofen der Glasfaserproduktionsanlage zugeführt. Die Feststoffzuführung kann separat oder im Gemisch mit dem Hauptdosierstrom der Glasrohstoffmischung erfolgen.

Ebenfalls bevorzugt wird Verfahrensschritt b) in unmittelbarer räumlicher Nähe zu einer Glasfaser-Produktionsanlage durchgeführt und die aus Verfahrensschritt b) resultierende Glasschmelze direkt mit der Glasschmelze der Glasfaserproduktionsanlage vereinigt.

Im Falle, dass es sich bei der Glas basierten Komponente des GFK um Glasfasern handelt, werden während des Verbrennungsvorgangs im Verfahrensschritt b) auch Verunreinigungen auf der Oberfläche der Glasfaser oder Schlichten oxidiert und über die Verbrennungsgase ausgetragen, vorzugsweise in Form von CO₂.

Vorzugsweise werden Verunreinigungen oder Abbauprodukte aus der Polymermatrix des eingesetzten GFK, die sich im Verfahrensschritt b) nicht zu CO₂ oxidieren lassen, über die Verbrennungsgase ausgetragen.

Verunreinigungen, Additive oder deren Abbauprodukte können im Verfahrensschritt b) Schadstoffe bilden. Diese werden vorzugsweise zusammen mit den anfallenden Verbrennungsgasen einer Abgasreinigung zugeführt, wo die Schadstoffe abgefangen werden um die gesetzlichen Regelungen des Immissionsschutzes einzuhalten.

Insbesondere bei einer in Verfahrensschritt b) erfolgenden Verbrennung eines Brom- oder Phosphor-haltige Additiv-Reste enthaltenden (Matrix)Polymers ist es prinzipiell möglich, dass für Mensch und Umwelt ungewollte toxische Nebenprodukte entstehen, die in den Verbrennungsgasen enthalten sind. Mit Hilfe einer modernen Abgasreinigung ist es aber heute problemlos möglich, diese aus dem Abgas abzutrennen, so dass diese Stoffe unter Einhaltung gesetzlicher Emissionsauflagen nicht in die Umwelt gelangen. Das deutsche Bundes-Immissionsschutzgesetz (BImSchG = Gesetz zum Schutz vor schädlichen Umwelteinwirkungen durch Luftverunreinigungen, Geräusche, Erschütterungen und ähnliche Vorgänge) regelt insbesondere in seiner neuesten Fassung vom 12. April 2019 (BGBI. I S. 432 ) ein wichtiges Teilgebiet des Umweltrechts und ist das praxisrelevante Regelwerk zum Schutz von Menschen, Tieren, Pflanzen, Böden, Wasser, Atmosphäre und Kulturgütern vor Immissionen und Emissionen.

In einer Verfahrensvariante des Verfahrensschrittes b) wird den Glas basierten Komponenten, vorzugsweise Glasfasern, anhaftendes organisches (Matrix)Polymer bei erhöhter Temperatur zunächst pyrolysiert und dann die Verbrennungswärme der an den Glasbestandteilen bzw. Glasfasern anhaftenden Verkohlungsreste und die Verbrennungswärme des anfallenden Pyrolysegases und/oder Pyrolyseöls gemeinsam zum Aufschmelzen der Glasbestandteile bzw. Glasfasern genutzt.

Besonders bevorzugt ist es, in Verfahrensschritt b) anfallende Pyrolysegase an anderer Stelle des Aufschmelzvorgangs im erfindungsgemäßen Verfahren einzuspeisen. Bevorzugt ist es ebenfalls, die Pyrolysegase direkt mit dem für den Aufschmelzvorgang im Verfahrensschritt b) vorzugsweise einzusetzenden Erdgas zu mischen. Mit dieser Verfahrensvariante ist es möglich, selbst nach einer vollständigen Depolymerisation des (Matrix)Polymers im Verfahrensschritt a) noch genügend Verbrennungswärme im Verfahrensschritt b) bereitzustellen, um die Glas basierte Komponente, vorzugsweise Glasfasern, aufzuschmelzen und bis zur weiteren Verarbeitung auf Schmelztemperatur zu halten.

Im Falle, dass Verfahrensschritt b) direkt und unmittelbar im Aufschmelzbereich einer Glasfaserproduktionsanlage erfolgt, werden vorzugsweise konventionelle Glasfaserzuschlagstoffe, insbesondere SiO₂, Al₂O₃, MgO, B₂O₃, CaO, in die aus Verfahrensschritt a) anfallende Masse aus Glas/Matrixresten eingetragen.

### Verfahrensschritt c)

Sofern Verfahrensschritt b) nicht bereits in oder in der räumlichen Nähe eines Ofens zur Glasproduktion durchgeführt wird, erfolgt in einem Verfahrensschritt c) die Abtrennung der Glasschmelze für eine spätere Weiterverarbeitung.

Vorzugsweise wird die im Verfahrensschritt c) anfallende Glasschmelze einer Herstellung von Glasfasern oder einer Herstellung von Glaspulvern oder Glaskugeln zugeführt. Besonders bevorzugt wird die in Verfahrensschritt c) anfallende Glaskomponente einem konventionell betriebenen Ofen zur Produktion von Glasfasern zugeführt, um dann dem erneuten Verspinnen zu Glasfasern zur Verfügung zu stehen.

Da sich nahezu alle Verunreinigungen im organischen Rückstand am Ende des Verfahrensschrittes a) konzentrieren und über den sich anschließenden Verbrennungsprozess im Verfahrensschritt b) in Form von Verbrennungsgasen ausgetragen und damit von den Glasbestandteilen entfernt werden, ermöglicht das erfindungsgemäße Verfahren die Herstellung einer hochwertigen Glasschmelze, aus der wiederum hochwertige Glasrezyklate, insbesondere Glasrezyklate in Form von Glasfasern, Mahlglas oder Glaspulver, in weiteren Verarbeitungsschritten hergestellt werden können.

Im bevorzugten Fall der Depolymerisation bzw. Pyrolyse der Polymermatrix in Verfahrensschritt a) bis maximal 80 Gew.-% der ursprünglichen GFK Matrix verbleibt noch genügend organisches Material auf den Glasbestandteilen, dass dessen Verbrennung eine ausreichende Verbrennungswärmemenge liefert, um die Glas basierte Komponente des GFK, vorzugsweise Glasfasern, damit aufzuschmelzen und einem werkstofflichen Recycling zuzuführen. Mit den vom ursprünglichen (Matrix)Polymer verbleibenden maximal 20 Gew.-% organischen Materials werden auf der Glas basierten Komponente verbleibende Verunreinigungen, Additive und Zersetzungsprodukte beim Verbrennungsprozess zu CO₂ oxidiert und auf diese Weise entfernt.

### Insbesondere bevorzugte Ausführungsformen der Erfindung

Insbesondere betrifft die vorliegende Erfindung ein Verfahren zum Rezyklieren von GFK, durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile, und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte mit der Maßgabe, dass die Polymermatrix auf Polyamid 6 (PA6), auf Polyamid 66 (PA66), auf Polybutylenterephthalat (PBT) oder auf einem Mischpolymer aus PBT und PET basiert.

Insbesondere betrifft die vorliegende Erfindung zudem ein Verfahren zum Rezyklieren von GFK, durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte mit der Maßgabe, dass die Polymermatrix auf Polyamid 6 (PA6) oder auf Polyamid 66 (PA66), insbesondere PA6, basiert.

Insbesondere betrifft die vorliegende Erfindung auch ein Verfahren zum Rezyklieren von GFK, durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte, mit der Maßgabe, dass die Polymermatrix auf Polybutylenterephthalat (PBT) oder aus einem Mischpolymer aus PBT und PET basiert.

Insbesondere betrifft die vorliegende Erfindung auch ein Verfahren zum Rezyklieren von GFK, durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte, und
c) Abtrennung der Glasschmelze für eine Weiterverarbeitung
mit der Maßgabe, dass die Polymermatrix auf Polyamid 6 (PA6), auf Polyamid 66 (PA66), auf Polybutylenterephthalat (PBT) oder auf einem Mischpolymer aus PBT und PET basiert.

Insbesondere betrifft die vorliegende Erfindung ferner ein Verfahren zum Rezyklieren von GFK, durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte, und
c) Abtrennung der Glasschmelze für eine Weiterverarbeitung
mit der Maßgabe, dass die Polymermatrix auf Polyamid 6 (PA6) oder auf Polyamid 66 (PA66), insbesondere PA6, basiert.

Insbesondere betrifft die vorliegende Erfindung schließlich ein Verfahren zum Rezyklieren von GFK, durch
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte, und
c) Abtrennung der Glasschmelze für eine Weiterverarbeitung
mit der Maßgabe, dass die Polymermatrix auf Polybutylenterephthalat (PBT) oder aus einem Mischpolymer aus PBT und PET basiert.

### Beispiele

150 g geschredderter GFK aus Durethan^{®} BKV30H2.0 der Lanxess Deutschland GmbH wurden in einer Glasapparatur mit KPG Rührer (Blattrührer & Drehmomentmessung) in einem 500 mL Rundkolben in einem Metallbad (T=320°C) aufgeschmolzen. 5 % Kaliumcarbonat, bezogen auf den Altkunststoff, wurde als fein pulverisierter Depolymerisations-Katalysator zugegeben.

Der Aufschmelzprozess wurde statisch durchgeführt und nur periodisch (ca. alle 5 min) mit etwa 2 bis 3 Umdrehungen umgerührt. Das Aufschmelzen der 150 g geschredderten GFKs war nach ca. 40 Minuten abgeschlossen.

Unter langsamem Rühren mit 12 Umdrehungen pro Minute (rpm) wurde in 50 mbar Schritten der Innendruck auf 20 bis 30 mbar abgesenkt und eine erhebliche Schaumentwicklung beobachtet.

Das Polyamid 6 Edukt Caprolactam wurde dabei in den gasförmigen Zustand überführt und die gesamte Apparatur mittels Heißluftfön kontinuierlich beheizt, damit dieses Monomer mit einem Schmelzpunkt von 68°C nicht in den festen Aggregatzustand überging und Verstopfungen in der Apparatur verursachte.

Folgende in Tab. 1 dargestellte Fraktionen Caprolactam wurden gewonnen:

**Tab. 1**

| KOLBEN | Dauer (min) | Menge (g) |
|---|---|---|
| 1 | 32 | 43,14 |
| 2 | 25 | 40,34 |
| 3 | 44 | 39,42 |
| Summe | 101 | 82,90 |
| Ausbeute | | 79% |

Die Caprolactam-Fraktionen 1 bis 3 wurden mittels Gaschromatograph analysiert. Die Reinheit nahm von Fraktion 1 bis zur Fraktion 3 ab, erwies sich aber in jedem Fall als geeignet, um mit Hilfe einer hydrolytischen Polymerisation re-polymerisiert zu werden. Der Caprolactam-Anteil in diesen Fraktionen lag über 99.5 Gew.-% !

Der nach der Depolymerisation verbliebene Rückstand wurde portioniert und in ein Glas-Schiffchen überführt, dort in einem Muffelofen mit reinem Sauerstoff umspült, mit Hilfe eines Bunsenbrenners gezündet und ohne weitere Wärmezuführung verbrannt.

Am Ende des Verbrennungsvorganges wurde der Glasrückstand isoliert, nach DIN 52331 bei 115°C getrocknet und homogenisiert. Anschließend wurden die Proben einer ICP-OES-Untersuchung unterzogen.

Bis auf eine gemessene CuO Konzentration, die auf der Wärmestabilisierung des eingesetzten Durethan^{®} Grades zurück zu führen war, zeigten sich keine auffälligen Abweichungen in der Glaszusammensetzung des Glasrückstands im Vergleich zu der in Durethan^{®} BKV30H2.0 eingesetzten Glasfaser.

## Patentansprüche

1. Verfahren zum Rezyklieren von GFK, **gekennzeichnet durch**
a) Depolymerisieren von bis zu 80 Gew.-% der Polymermatrix eines GFK, Abtrennen der aus der Polymermatrix entstehenden Spaltprodukte und Anreichern der verbleibenden Rückstände in einem Gemisch aus Glas basierter Komponente, Restmatrix, Monomer, Spaltprodukten sowie der für ein Recycling problematischen Bestandteile und
b) Nutzen des am Ende von Verfahrensschritt a) im Rückstand verbleibenden organischen Anteils als Energielieferanten unter Verwendung von dessen Verbrennungswärme zum Erwärmen und Aufschmelzen der Glas basierten Komponente sowie gleichzeitig zum Entfernen der organischen Bestandteile durch Umwandlung in gasförmige Verbrennungsprodukte
mit der Maßgabe, dass die Polymermatrix eines im erfindungsgemäßen Verfahren einzusetzenden GFK im Wesentlichen wenigstens ein Polymer enthält aus der Gruppe Polyethlenterephthalat (PET), Polybutylenterephthalat (PBT) oder ein Mischpolymer aus PET und PBT.

2. Verfahren gemäß Anspruch 1, **dadurch gekennzeichnet, dass** mindestens 50 Gew.-% der Polymermatrix, vorzugsweise mindestens 50 Gew.-% und höchstens 80 Gew.-% der Polymermatrix, depolymerisiert werden.

3. Verfahren gemäß Anspruch 1 oder 2, mit der Maßgabe, dass die Polymermatrix auf Polybutylenterephthalat (PBT) oder auf einem Mischpolymer aus PBT und PET basiert

4. Verfahren gemäß einem oder mehrerer der Ansprüche 1 bis 3, **dadurch gekennzeichnet, dass** die in Verfahrensschritt a) einzusetzenden GFK Komponenten zuvor sortenähnlich, bevorzugt sortenrein, gesammelt und sortenähnlich, vorzugsweise sortenrein in Verfahrensschritt a) eingesetzt werden.

5. Verfahren gemäß einem oder mehrerer der Ansprüche 1 bis 4, **dadurch gekennzeichnet, dass** die in Verfahrensschritt a) einzusetzenden GFK Komponenten vor dem Einsatz in der Depolymerisation zu Kleinteilen geschreddert werden.

6. Verfahren gemäß einem oder mehrerer der Ansprüche 1 bis 5, **dadurch gekennzeichnet, dass** die in Verfahrensschritt a) einzusetzenden GFK vor der Depolymerisation einer Reinigung unterzogen werden.

7. Verfahren gemäß einem oder mehrerer der Ansprüche 1 bis 6, **dadurch gekennzeichnet, dass** in Verfahrensschritt a) einzusetzende GFK in Gegenwart von Wasser oder Alkoholen depolymerisiert werden.

8. Verfahren gemäß einem oder mehrerer der Ansprüche 1 bis 7, **dadurch gekennzeichnet, dass** während oder im Anschluss an Verfahrensschritt a) durch Reduzierung des Druckes die Spaltprodukte und/oder die zugesetzten Hydrolyse bzw. Solvolyse Flüssigkeiten abdestilliert werden.

9. Verfahren gemäß einem oder mehrerer der Ansprüche 1 bis 8, **dadurch gekennzeichnet, dass** in Verfahrensschritt a) wenigstens 20 Gew.-% der ursprünglichen Polymermatrix im organischen Rückstand verbleiben.

10. Verfahren gemäß einem oder mehrerer der Ansprüche 1 bis 9, **dadurch gekennzeichnet, dass** die in Verfahrensschritt a) unter Spaltung der Polymerketten anfallenden Spaltprodukte des GFK (Matrix)Polymers einer sich anschließenden erneuten Polymerisation zur Herstellung von Rezyklat-Kunststoff mit Neuwarencharakter zugeführt werden.

11. Verfahren gemäß einem oder mehrerer der Ansprüche 1 bis 10, **dadurch gekennzeichnet, dass** es sich bei dem Spaltprodukten um Terephthalsäure und 1,4 Butandiol bzw. dessen Dehydrationsprodukt Tetrahydrofuran und bei dem Rezyklat-Kunststoff um Polybutylenterephthalat handelt.

12. Verfahren gemäß einem oder mehrerer der Ansprüche 1 bis 11, **dadurch gekennzeichnet, dass** die in Verfahrensschritt a) unter Spaltung der Polymerketten anfallenden Spaltprodukte des GFK (Matrix)Polymers zumindest zu einem Teil auch als Brennstoff für den Verbrennungsvorgang im Verfahrensschritt b) eingesetzt werden.

13. Verfahren gemäß einem oder mehrerer der Ansprüche 1 bis 12, **dadurch gekennzeichnet, dass** mit den verbleibenden wenigstens 20 Gew.-% organischen Materials auf den Glasbestandteilen verbleibende Verunreinigungen, Additive und Zersetzungsprodukte beim Verbrennungsprozess in Verfahrensschritt b) von der Glasverstärkung/Glasschmelze entfernt werden, vorzugsweise durch Oxidation zu CO₂ umgewandelt werden.

14. Verfahren gemäß einem oder mehrerer der Ansprüche 1 bis 14, **dadurch gekennzeichnet, dass** in Verfahrensschritt b) zusätzliche Energie zugeführt wird, bevorzugt mittels Gasbrenner.
